# EUROPEAN PATENT SPECIFICATION

(11) **EP 2 405 892 B1**
(45) Date of publication and mention of the grant of the patent: **16.01.2013**
(21) Application number: 10701698.2
(22) Date of filing: 02.02.2010
(51) Int. Cl.: A61K 9/08, A61K 31/192, A61K 47/10, A61K 47/14, A61K 9/70, A61K 9/06, A61K 9/12

(54) **PHARMACEUTICAL FORMULATION COMPRISING KETOPROFEN AND A POLYOXYALKYLENE ESTER OF A HYDROXY FATTY ACID**
PHARMAZEUTISCHE FORMULIERUNG MIT KETOPROFEN UND EINEM POLYOXYALKYLENESTER EINER HYDROXYFETTSÄURE
FORMULATION PHARMACEUTIQUE COMPRENANT DU KÉTOPROFÈNE ET UN ESTER DE POLYOXYALKYLÈNE D'UN HYDROXY-ACIDE GRAS

(30) Priority: 12.03.2009 EP 09155046
(43) Date of publication of application: 18.01.2012
(73) Proprietor: Advance Holdings Limited, Floriana (MT)
(72) Inventor: VIRNO, Michele, I-00136 Roma (IT)
(74) Representative: Allaix, Roberto
(86) International application number: PCT/EP2010/051222
(87) International publication number: WO 2010/102862

(56) References cited:
- EP-A- 0 756 870
- WO-A-01/12229
- US-A- 4 917 886

## Description

### FIELD OF THE INVENTION

The present invention relates to a pharmaceutical formulation comprising ketoprofen.

More in particular, the present invention relates to a pharmaceutical formulation comprising ketoprofen for topical application having improved permeation and bioavailability properties.

### BACKGROUND OF THE INVENTION

Ketoprofen is a member of the arylpropionic acid group of non-steroidal anti-inflammatory drug ("NSAID"), with the chemical name of 2-(3-benzoylphenyl) propionic acid (CAS RN: 22071-15-4) having remarkable analgesic, anti-inflammatory, antipyretic and anti-rheumatic properties. The usual dosage for ketoprofen, which is normally administered in acid form, is of two daily 100 mg doses or three daily 50 mg doses. The exact metabolism of ketoprofen is unknown but, however, it has been ascertained that it is also extensively metabolized in the liver.

Ketoprofen was introduced in 1986 and has gained wide acceptance and is used for the relief of signs and symptoms of rheumatoid arthritis and osteoarthritis and for the treatment of dysmenorrhea. Ketoprofen is used alone or as an adjunct in the treatment of acute biliary colic, pain due to renal colic, pain associated with oral surgery, severe postpartum pain and for fever. (PDR Generics, 1996, second edition, Medical Economics, Montvale, New Jersey, pg 1812). Ketoprofen may be used for bone regeneration (Alfano, M.C.; Troullos, E.S., US Patent No. 5,902,110).

It is very difficult, however, to deliver therapeutically effective plasma levels of these kind drugs into the host by formulation, due to the slow skin penetration rate.

Ketoprofen is generally taken orally in the form of normal tablets or tablets covered with coatings resistant to gastric juices, or rectally, or by injection, or topically. Oral administration of ketoprofen can cause serious adverse effects most notably gastrointestinal disturbances such as dyspepsia, gastroduodenal bleeding, gastric ulcerations, and gastritis, particularly after extended use.

Therefore, in an effort to minimize the adverse effects associated with oral administration, non-oral delivery of ketoprofen has been extensively investigated in recent years.

Topical formulations are attractive options because they avoid the hepatic first-pass metabolism, reduce the side effects associated with oral administration, are associated with higher patient compliance and, in some cases, enhance therapeutic efficacy of the drug.

However, the effectiveness of topical administration of ketoprofen is limited by the difficulty of this drug to permeate the skin and by its low solubility in water.

Several patents and patent applications attempted to solve the above mentioned problems by means of topical formulations containing several ingredients to improve the permeation of ketoprofen.

US 5,654,337 discloses a pharmaceutical composition for the delivery of a NSAID like ibuprofen or ketoprofen through the skin which comprises a biocompatible organic solvent, a polar lipid, a surfactant, water, urea and the NSAID to be delivered, at a pH of about 6.0 to 8.0, wherein the polar lipid is lecithin or phosphatidylcholine, the biocompatible organic solvent is an isopropyl ester, selected from the group consisting of isopropyl myristate and isopropyl palmitate, and the urea is present at a concentration of about 5 to 20% by mass of the final composition.

US 5,885,597 discloses a topical composition comprising an arylpropionic acid type analgesic, like ibuprofen or ketoprofen, and an amount effective to increase the skin permeation of at least one phospholipid, such a lecithin, and at least one polyoxyethylenepolyoxypropylene copolymer.

US 6,083,996 discloses an aqueous pharmaceutical composition of a semisolid consistency for topical application of NSAIDs, like ketoprofen, comprising (i) a non-basic polymeric skin penetration enhancer selected from a water-dispersible acid polymer, a polysaccharide gum, and a mixture thereof and (ii) a lipophilic solvent mixture of an aliphatic C₂ to C₈ alcohol and an aliphatic C₈ to C₃₀ ester.

US 6,759,056 and US 6,946,144 disclose a transdermal delivery system comprising a mixture of an NSAID such as ketoprofen, an ethoxylated oil, an alcohol and an aqueous adjuvant such as water or Aloe Vera extracts.

EP 756 870 describes and claims a pharmaceutical spray composition for topic use comprising an hydroalcoholic solution of ketoprofen, propylene glycol, poloxamers or polyoxyethylated castor oil, polyvinylpyrrolidone, and ethanolamine. Castor oil is a vegetable triglyceride oil comprising unsaturated fatty acids, in which approximately ninety percent of fatty acid chains are ricinoleic acid, with minor but significant amounts of oleic and linoleic acids. Polyoxyethylated castor oil is the reaction product of castor oil with ethylene oxide, wherein the hydroxyl groups of the castor oil triglyceride have been ethoxylated with ethylene oxide to form polyethylene glycol ethers. Polyoxyethylated castor oil is commercially available under the tradename Cremophor EL from BASF Corp.

EP 707 847 discloses a ketoprofen liposome gel and a process for its preparation which is distinguished by a simple method of preparation by spontaneous formation of ketoprofen containing liposomes from phospholipids or non-ionic amphiphiles (such as Cremophor RH 40, a polyoxyethylated hydrogenated castor oil commercially available from BASF Corp).

A gel topical formulation comprising ketoprofen is sold throughout Europe by Cyathus Exquirere PharmaforschungsGmbH under the tradename Ketospray^{™}. The formulation comprises soybean lecithin as solubilizer and alcohols and glycols as co-solvents. Further details on the formulation can be found in US 5,958,379, which discloses a sprayable liquid pharmaceutical composition containing at least one active substance, at least one gel-forming agent consisting of a phospholipid or a phospholipid mixture, an alcohol or an alcohol mixture easily vaporizable, and water.

### SUMMARY OF THE INVENTION

The Applicant has perceived that in spite of the several efforts made in the art, there is still the need to develop a pharmaceutical formulation for the topical administration of ketoprofen having improved permeation and bioavailability properties.

The Applicant has also perceived that there is still the need of a pharmaceutical formulation for the topical administration of ketoprofen comprising a high concentration of ketoprofen, such as, for example, higher than 5% w/v, and even as high as 10% w/v or more, in the form of a liquid formulation able to be sprayed and/or nebulized on the skin and/or mucous surface to be treated.

The Applicant has found that the above mentioned problems may be overcome by a pharmaceutical formulation comprising (i) ketoprofen, (ii) at least one polyoxyalkylene ester of a hydroxy fatty acid, (iii) at least one hydroxy substituted organic compound selected from the group consisting of alcohols, polyols or a mixture thereof, and (iv) water.

The Applicant has surprisingly found that the pharmaceutical formulation of the present invention has improved permeation and bioavailability properties.

Moreover, the Applicant has surprisingly found that the pharmaceutical formulation of the present invention allows to reduce the formation of precipitate when stored at low temperature near to 0°C.

### SHORT DESCRIPTION OF THE FIGURES

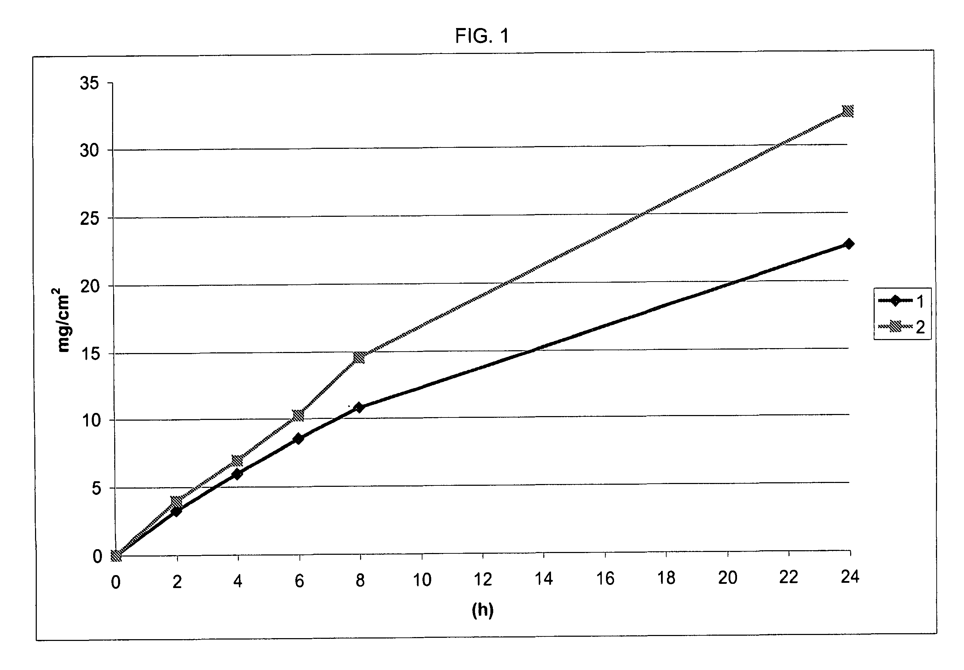

Figure 1 shows the diffusion profiles of the pharmaceutical formulations 1 (line 1) and 2 (line 2) described in the Examples. The ordinate values represent the permeated cumulative amount expressed in milligram per square centimeter (mg/cm²), the abscissa values represent the elapsed time expressed in hours (h).

### DETAILED DESCRIPTION OF THE INVENTION

The pharmaceutical formulation of the present invention may show one or more of the preferred characteristics hereinafter described.

The concentration of ketoprofen in the pharmaceutical formulation of the present invention is preferably between 2% and 15% (w/v), more preferably between 5% and 10% (w/v). Advantageously, the concentration of ketoprofen in the pharmaceutical formulation of the present invention is about 10% (w/v).

Preferably, said at least one polyoxyalkylene ester of a hydroxy fatty acid is obtained from the esterification of a hydroxy fatty acid having from 8 to 30 carbon atoms, preferably from 14 to 24 carbon atoms, with a polyoxyalkylene having a molecular weight ranging from 200 to 6,000, preferably from 400 to 1,500.

Advantageously, said hydroxy fatty acids are selected from the group comprising saturated chains, such as hydroxycaprylic acid, hydroxycapric acid, hydroxylauric acid, hydroxymyristic acid, hydroxypalmitic acid, hydroxystearic acid, hydroxyarachidic acid, hydroxybehenic acid, hydroxylignoceric acid, and unsaturated chains, such as hydroxymyristoleic acid, hydroxypalmitoleic acid, hydroxyoleic acid, hydroxylinoleic acid, hydroxylinolenic acid, hydroxyarachidonic acid, hydroxyeicosapentaenoic acid, hydroxyerucic acid, and hydroxydocosahexaenoic acid.

Particularly useful hydroxy fatty acid are selected from the group of saturated hydroxy fatty acids comprising hydroxylauric acid, hydroxymyristic acid, hydroxypalmitic acid, hydroxystearic acid, and hydroxyarachidic acid. The Applicant has found that the use of saturated hydroxy fatty acids is preferable, because the presence of unsaturation in the fatty acid chains can favor the degradation by oxidation and reduce the lifetime of the pharmaceutical formulation. A particularly preferred hydroxy fatty acid is hydroxystearic acid.

Advantageously, said polyoxyalkylene is selected from the group comprising polyethylene glycol 200 (PEG 200), polyethylene glycol 300 (PEG 300), polyethylene glycol 400 (PEG 400), polyethylene glycol 600 (PEG 600), polyethylene glycol 660 (PEG 660), polyethylene glycol 1000 (PEG 1000), polyethylene glycol 1500 (PEG 1500), polyethylene glycol 3000 (PEG 3000), polyethylene glycol 3350 (PEG 3350), polyethylene glycol 4000 (PEG 4000), polyethylene glycol 6000 (PEG 6000), and mixtures thereof.

According to a preferred embodiment, said polyoxyalkylene comprises polyethylene glycol 400 (PEG 400), polyethylene glycol 600 (PEG 600), polyethylene glycol 660 (PEG 660), polyethylene glycol 1000 (PEG 1000), polyethylene glycol 1500 (PEG 1500), and mixtures thereof.

According to a preferred embodiment of the present invention said at least one polyoxyalkylene ester of a hydroxy fatty acid is selected from the group of Solutol™ HS 15 (polyethylene glycol 660 hydroxy stearate - Ph. Eur. Name: Macrogol 15 Hydroxystearate), a polyglycol ester of polyethylene glycol and 12-hydroxystearic acid, and mixtures thereof.

Solutol HS 15 is a polyethylene glycol 660 hydroxystearate manufactured by BASF (Parsippany, N.J.). Apart from free polyethylene glycol and its monoesters, diesters are also detectable. According to the manufacturer, a typical lot of Solutol HS 15 contains approximately 30% free polyethylene glycol and 70% polyethylene glycol esters.

The concentration of said at least one polyoxyalkylene ester of a hydroxy fatty acid in the pharmaceutical formulation of the present invention is preferably from 1% to 20% (w/v), more preferably from 2% to 15% (w/v), and most preferably from 5% to 15% (w/v). Advantageously, the concentration of the polyoxyalkylene hydroxy fatty acid ester is from about 5% to about 10% (w/v).

Advantageously, the pharmaceutical formulation of the present invention comprises at least one alcohol, or at least one polyol or a mixture thereof.

Preferably, said at least one alcohol is selected from the group comprising pharmaceutically acceptable alcohols, such as for example, ethanol, 1-propanol, 2-propanol, and mixture thereof.

Preferably, said at least one polyol is selected from the group comprising pharmaceutically acceptable polyols, such as for example, glycerol, propylen glycol, 1,3-butylene glycol, and mixtures thereof.

Advantageously, the pharmaceutical formulation of the present invention comprises a mixture of 2-propanol and glycerol. More preferably, the pharmaceutical formulation of the present invention comprises a mixture of 2-propanol and propylene glycol.

The concentration of said at least one alcohol in the pharmaceutical formulation of the present invention is preferably from 1% to 30% (w/v), more preferably from 2% to 20% (w/v).

The concentration of said at least one polyol in the pharmaceutical formulation of the present invention is preferably from 1% to 30% (w/v), more preferably from 2% to 20% (w/v).

Advantageously, the pharmaceutical formulation of the present invention comprises a mixture of at least one alcohol and at least one polyol. When mixed together, the total concentration of said at least one alcohol and said at least one polyol in the pharmaceutical formulation of the present invention is preferably from 1% to 30% (w/v), more preferably from 10% to 30% (w/v). In such a case, advantageously, the concentration of said at least one alcohol ranges from 5% to 15% (w/v) and the concentration of said at least one polyol ranges from 5% to 15% (w/v).

The pH of the pharmaceutical formulation of the present invention is preferably ranging from 6.5 to 8.5, more preferably from 7.0 to 8.0. Advantageously, the pH of the pharmaceutical formulation of the present invention is ranging from 7.3 to 7.6.

The pharmaceutical formulation of the present invention may further comprise several additives generally known and used in the art. Such non-essential additives of the pharmaceutical formulation according to the invention are, for example, stabilizers, antioxidants, pH correctors, buffers, surfactants, colorants and/or perfumes.

The pharmaceutical formulation according to the present invention can be formulated into a preparation form which is commonly employed as a preparation form for topical application. Advantageously useful preparation forms include, but are not limited specifically to, various solutions, ointments, creams, sprays, foams, cataplasm plasters, and the like. Topical preparations in the form of solution and spray are particularly preferred.

The pharmaceutical formulation of the present invention can be used as analgesic for the treatment of various types of pain, including both chronic and acute painful episodes, such as, for examples, for the treatment of musculoskeletal and joint disorders, like rheumatoid arthritis, osteoarthritis, and ankylosing spondylitis; periarticular disorders, like bursitis and tendonitis; soft tissue disorders, like sprains and strains; and other painful conditions like those following some surgical procedures.

The following examples will illustrate at least one way of carrying out the invention, without however in any way restricting the matter for which protection is sought which is defined by the annexed claims.

### Example 1

The reference pharmaceutical formulation 1 was a commercial pharmaceutical formulation sold by Cyathus Exquirere PharmaforschungsGmbH under the trade name Ketospray™.

The pharmaceutical formulation 2 according to the invention contained the ingredients of the following Table 1.

**TABLE 1**

| Ingredient | Amount |
|---|---|
| Ketoprofen | 10.0 g |
| Solutol HS 15 | 7.5 g |
| Propylene glycol | 9.6 ml |
| Isopropyl alcohol | 13.3 ml |
| NaH₂PO₄ * H₂O | 0.25 |
| Na₂HPO₄ * 12H₂O | 0.57 |
| NaOH 10 N | 3.9 ml |
| Peppermint oil | 0.15 ml |
| Purified water | q.s. 100 ml |
| Final pH | 7.4 |

The pharmaceutical formulation 2 was prepared as follows.

Into a vessel provided with a magnetic stirring ketoprofen, Solutol HS 15, propylene glycol, and isopropyl alcohol were introduced. The mixture was stirred for about 20 minutes obtaining a homogenous mass (mixture A).

Into a separate vessel provided with a magnetic stirring NaH₂PO₄ * H₂O, Na₂HPO₄ * 12H₂O, and water were introduced. The mixture was stirred for about 20 minutes obtaining a clear colorless solution (mixture B).

Then, mixture B was slowly added under stirring to mixture A. Stirring was continued for about 10 minutes obtaining a milky solution having a pH of about 4.7. The pH was corrected to 7.4 with NaOH 10N to give a clear solution. Finally, peppermint oil was added to the solution and the volume was brought to 100 ml with purified water.

For comparison of the skin permeation of ketoprofen standard diffusion experiments with Franz type diffusion cells through porcine skin were performed.

### HPLC analysis

For the quantification of ketoprofen the method described in C. Valenta, M. Wanka, and J. Heidlas, "Evaluation of novel-soja lecithin formulations for dermal use containing ketoprofen as a model drug", J. Contr. Rel. 63, 165-173 (2000) was used.

Analysis was done by HPLC (Perkin Elmer, US) consisting of an automatic autosampler ISS-200, a pump and an UV-diode array detector. The column (240 mm x 4 mm) packed with Nucelosil 100 - 5C18 was eluted at 45°C with a mobile phase consisting of acetonitrile and phosphate buffer, pH 5 (40:60 v/v) at a flow rate of 1.0 ml/min. The concentration of ketoprofen was established by comparing the peak area of the unknown with a standard calibration curve. Standard solutions contained between 0.052 and 0.0065 mg/ml ketoprofen. Linear regression analysis of the peak areas gave a correlation coefficient of 0.9965. Samples (20 µl) were withdrawn from the receptor chamber and injected directly by the autosampler.

### Parameters

Stock solution: 1.042 mg/ml methanol
Column: Nucleosil C18
Mobile phase: acetonitrile/phosphate-buffer pH=5 (40:60/ v:v)
UV-detection λmax : 260 nm
Flow rate: 1.0 ml/min
Retention time about 5.5 min

### Skin preparation

Porcine abdominal skin was shaved and then prepared with a dermatome (GB 228R, Aesculap) set at 1.1 mm. The skin was stored in a freezer at -20°C until use. Two hours prior to the experiment the samples were thawed.

### Diffusion cell preparation

Standard diffusion experiments using Franz-type diffusion cells (Permegear, USA) with about 1 cm² of permeation area and porcine skin were performed. The receptor compartment was filled with 2 ml of 0.012 M phosphate buffer (pH 7.4). Excised skin was mounted in the cell, stratum corneum uppermost, with the dermal side facing the receptor compartment. The diffusion cells were thermostated at skin surface temperature of 32°C and stirred by magnetic bars. At defined time intervals (2, 4, 6, 8 and 24 h) the acceptor medium was removed for analysis and replaced with fresh acceptor medium. If necessary, a suitable dilution with acceptor medium was additionally performed. Each amount of applied formulation was equivalent to 65 mg ketoprofen. Three parallel tests were performed for each product. The following Tables and Fig. 1 illustrate the average results and standard deviation of the tests.

**TABLE 2**

| Formulation 1 (Reference) | | | | |
|---|---|---|---|---|
| Hours | Average (mg/cm²) | Standard Deviation | Average (%) | Standard Deviation |
| 0 | n.a. | n.a. | n.a. | n.a. |
| 2 | 3.292 | 0.392 | 5.117 | 0.643 |
| 4 | 5.957 | 1.564 | 9.283 | 2.537 |
| 6 | 8.521 | 2.508 | 13.291 | 4.078 |
| 8 | 10.768 | 2.689 | 16.790 | 4.461 |
| 24 | 22.685 | 2.756 | 35.305 | 4.938 |

**TABLE 3**

| Formulation 2 (Invention) | | | | |
|---|---|---|---|---|
| Hours | Average (mg/cm²) | Standard Deviation | Average (%) | Standard Deviation |
| 0 | n.a. | n.a. | n.a. | n.a. |
| 2 | 3.988 | 1.737 | 6.215 | 2.735 |
| 4 | 6.948 | 0.671 | 10.798 | 0.984 |
| 6 | 10.243 | 0.890 | 15.909 | 1.110 |
| 8 | 14.550 | 1.919 | 22.588 | 2.629 |
| 24 | 32.460 | 2.230 | 50.426 | 2.630 |

The data of tables 2 and 3 clearly show that formulation 2 of the present invention has demonstrated an improved permeation, in particular after 8 and 24 hours of diffusion, where the amount of permeated ketoprofen of formulation 2 is about 140% the amount of reference formulation 1.

Two samples of 10 ml of pharmaceutical formulations 1 and 2 were stored at 4°C for promoting the precipitation. The resulting precipitate was filtered and weighted after dryness. Pharmaceutical formulation 1 gave 134 mg of precipitate, while pharmaceutical formulation 2 gave only 94 mg of precipitate with a reduction of about 30 % w/w.

### Example 2

The pharmaceutical formulations 3 to 7 contained the ingredients of the following Table 2.

**TABLE 2**

| | Formulation | | | | |
|---|---|---|---|---|---|
| Ingredient | 3 | 4 | 5 | 6 | 7 |
| Ketoprofen (g) | 10.0 | 10.0 | 10.0 | 10.0 | 10.0 |
| Solutol HS 15 (g) | 10.0 | 7.5 | 7.5 | 15.0 | 5.0 |
| Propylene glycol (ml) | 9.6 | 9.6 | 9.6 | 9.6 | 9.6 |
| Ethyl alcohol (ml) | - | - | 3.1 | 3.1 | 3.1 |
| Isopropyl alcohol (ml) | 13.3 | 13.3 | 13.3 | 13.3 | 13.3 |
| NaH₂PO₄ * H₂O (g) | 0.25 | 0.25 | 0.25 | 0.25 | 0.25 |
| Na₂HPO₄ * 12H₂O (g) | 0.57 | 0.57 | 0.57 | 0.57 | 0.57 |
| NaOH 10 N (ml) | 3.5 | 3.5 | 3.9 | 3.6 | 3.6 |
| Peppermint oil (ml) | 0.15 | 0.15 | 0.15 | 0.15 | 0.15 |
| Purified water (ml) | q.s. to 100 | | | | |
| Final pH | 7.4 | 7.4 | 7.4 | 7.6 | 7.4 |

The pharmaceutical formulations 3 to 7 were submitted to the same skin permeation evaluation of example 1 in comparison with pharmaceutical formulation 1. The results showed an improvement of skin permeation of pharmaceutical formulations 3 to 7 with respect to pharmaceutical formulation 1.

### Example 3

The pharmaceutical formulations 8 to 10 contained the ingredients of the following Table 3.

**TABLE 3**

| Ingredient | Formulation | | |
|---|---|---|---|
| | 8 | 9 | 10 |
| Ketoprofen (g) | 10.0 | 10.0 | 10.0 |
| Solutol HS 15 (g) | 7.5 | 7.5 | 7.5 |
| Propylene glycol (ml) | 9.6 | 19.8 | - |
| Ethyl alcohol (ml) | 13.0 | - | - |
| Isopropyl alcohol (ml) | - | - | 26.0 |
| NaH₂PO₄ * H₂O (g) | 0.25 | 0.25 | 0.25 |
| Na₂HPO₄ * 12H₂O (g) | 0.57 | 0.57 | 0.57 |
| NaOH 10 N (ml) | 2.0 | 3.9 | 3.9 |
| Peppermint oil (ml) | 0.15 | 0.15 | 0.15 |
| Purified water (ml) | q.s. to 100 | | |
| Final pH | 7.4 | 7.4 | 7.4 |

The pharmaceutical formulations 8 to 10 were submitted to the same skin permeation evaluation of example 1 in comparison with pharmaceutical formulation 1. The results showed an improvement of skin permeation of pharmaceutical formulations 8 to 10 with respect to pharmaceutical formulation 1.

## Claims

1. A pharmaceutical formulation comprising (i) ketoprofen, (ii) at least one polyoxyalkylene ester of a hydroxy fatty acid, (iii) at least one hydroxy substituted organic compound selected from the group consisting of at least one alcohol, at least one polyol and mixtures thereof, and (iv) water.

2. The pharmaceutical formulation according to claim 1, **characterized in that** said at least one polyoxyalkylene ester of a hydroxy fatty acid is obtained from the esterification of a hydroxy fatty acid having from 8 to 30 carbon atoms with a polyoxyalkylene having a molecular weight ranging from 200 to 6,000.

3. The pharmaceutical formulation according to claim 2, **characterized in that** said hydroxy fatty acid has from 14 to 24 carbon atoms, and said polyoxyalkylene has a molecular weight ranging from 400 to 1,500.

4. The pharmaceutical formulation according to any one of claims 2 and 3, **characterized in that** said hydroxy fatty acid is a saturated or unsaturated hydroxy fatty acid selected from the group consisting of hydroxycaprylic acid, hydroxycapric acid, hydroxylauric acid, hydroxymyristic acid, hydroxypalmitic acid, hydroxystearic acid, hydroxyarachidic acid, hydroxybehenic acid, hydroxylignoceric acid, hydroxymyristoleic acid, hydroxypalmitoleic acid, hydroxyoleic acid, hydroxylinoleic acid, hydroxylinolenic acid, hydroxyarachidonic acid, hydroxyeicosapentaenoic acid, hydroxyerucic acid, and hydroxydocosahexaenoic acid.

5. The pharmaceutical formulation according to claim 4, **characterized in that** said hydroxy fatty acid is a saturated hydroxy fatty acid selected from the group consisting of hydroxylauric acid, hydroxymyristic acid, hydroxypalmitic acid, hydroxystearic acid, and hydroxyarachidic acid.

6. The pharmaceutical formulation according to any one of claims 2 to 5, **characterized in that** said polyoxyalkylene is selected from the group consisting of polyethylene glycol 200 (PEG 200), polyethylene glycol 300 (PEG 300), polyethylene glycol 400 (PEG 400), polyethylene glycol 600 (PEG 600), polyethylene glycol 660 (PEG 660), polyethylene glycol 1000 (PEG 1000), polyethylene glycol 1500 (PEG 1500), polyethylene glycol 3000 (PEG 3000), polyethylene glycol 3350 (PEG 3350), polyethylene glycol 4000 (PEG 4000), polyethylene glycol 6000 (PEG 6000), and mixtures thereof.

7. The pharmaceutical formulation according to any one of claims 3 to 6, **characterized in that** said at least one polyoxyalkylene ester of a hydroxy fatty acid is selected from the group of Solutol™ HS 15 (polyethylene glycol 660 hydroxy stearate), polyglycol ester of polyethylene glycol and 12-hydroxystearic acid, and mixtures thereof.

8. The pharmaceutical formulation according to any one of preceding claims, **characterized in that** said at least one alcohol is selected from the group comprising ethanol, 1-propanol, 2-propanol, and mixture thereof.

9. The pharmaceutical formulation according to any one of preceding claims, **characterized in that** said at least one polyol is selected from the group comprising glycerol, propylen glycol, 1,3-butylene glycol, and mixtures thereof.

10. The pharmaceutical formulation according to any one of preceding claims, **characterized in that** said formulation comprises an amount of ketoprofen ranging from 2% to 15% (w/v), preferably between 5% and 10% (w/v), and more preferably of about 10% (w/v).

11. The pharmaceutical formulation according to any one of preceding claims, **characterized in that** said formulation comprises an amount of said at least one polyoxyalkylene ester of a hydroxy fatty acid ranging from 1% to 20% (w/v), preferably from 2% to 15% (w/v), more preferably from 5% to 15% (w/v), and most preferably from about 5% to about 10% (w/v).

12. The pharmaceutical formulation according to any one of preceding claims, **characterized in that** said formulation comprises an amount of said at least one alcohol ranging from 1% to 30% (w/v), preferably from 2% to 20% (w/v).

13. The pharmaceutical formulation according to any one of preceding claims, **characterized in that** said formulation comprises an amount of said at least one polyol ranging from 1% to 30% (w/v), preferably from 2% to 20% (w/v).

14. The pharmaceutical formulation according to any one of preceding claims, **characterized in that** said formulation comprises a mixture of at least one alcohol and at least one polyol, wherein the concentration of said at least one alcohol ranges from 5% to 15% (w/v) and the concentration of said at least one polyol ranges from 5% to 15% (w/v).

15. The pharmaceutical formulation according to any one of preceding claims, **characterized in that** said formulation has a pH value ranging from 6.5 to 8.5, preferably from 7.0 to 8.0.

## Patentansprüche

1. Pharmazeutische Formulierung, umfassend (i)-Ketoprofen, (ii) wenigstens einen Polyoxyalkylen-Ester einer Hydroxyfettsäure, (iii) wenigstens eine hydroxysubstituierte organische Verbindung, ausgewählt unter wenigstens einem Alkohol, wenigstens einem Polyol und Gemischen davon, und (iv) Wasser.

2. Pharmazeutische Formulierung nach Anspruch 1, **dadurch gekennzeichnet, daß** der wenigstens eine Polyoxyalkylen-Ester einer Hydroxyfettsäure durch Veresterung einer Hydroxyfettsäure mit 8 bis 30 Kohlenstoffatomen mit einem Polyoxyalkylenpolyol mit einem Molekulargewicht im Bereich von 200 bis 6.000 erhalten ist.

3. Pharmazeutische Formulierung nach Anspruch 2, **dadurch gekennzeichnet, daß** die Hydroxyfettsäure 14 bis 24 Kohlenstoffatome aufweist und das Polyoxyalkylen ein Molekulargewicht im Bereich von 400 bis 1.500 aufweist.

4. Pharmazeutische Formulierung nach einem der Ansprüche 2 und 3, **dadurch gekennzeichnet, dass** die Hydroxy-Fettsäure eine gesättigte oder ungesättigte Hydroxyfettsäure ist, die ausgewählt ist unter Hydroxycaprylsäure, Hydroxycaprinsäure, Hydroxylaurinsäure, Hydroxymyristinsäure, Hydroxypalmitinsäure, Hydroxystearinsäure, Hydroxyarachidinsäure, Hydroxybehensäure, Hydroxylignocerinsäure, Hydroxymyristoleinsäure, Hydroxypalmitoleinsäure, Hydroxyölsäure, Hydroxylinoleinsäure, Hydroxylinolensäure, Hydroxyarachidonsäure, Hydroxyeicosapentaensäure, Hydroxyerukasäure und Hydroxydocosahexaensäure.

5. Pharmazeutische Formulierung nach Anspruch 4, **dadurch gekennzeichnet, daß** die Hydroxyfettsäure eine gesättigte Hydroxyfettsäure ist, die ausgewählt ist unter Hydroxylaurinsäure, Hydroxymyristinsäure, Hydroxypalmitinsäure, Hydroxystearinsäure und Hydroxyarachidinsäure.

6. Pharmazeutische Formulierung nach einem der Ansprüche 2 bis 5, **dadurch gekennzeichnet, daß** das Polyoxyalkylen ausgewählt ist unter Polyethylenglykol 200 (PEG 200), Polyethylenglykol 300 (PEG 300), Polyethylenglykol 400 (PEG 400), Polyethylenglykol 600 (PEG 600), Polyethylenglykol 660 (PEG 660), Polyethylenglykol 1000 (PEG 1000), Polyethylenglykol 1500 (PEG 1500), Polyethylenglykol 3000 (PEG 3000), Polyethylenglykol 3350 (PEG 3350), Polyethylenglykol 4000 (PEG 4000), Polyethylenglycol 6000 (PEG 6000) sowie Gemischen davon.

7. Pharmazeutische Formulierung nach einem der Ansprüche 3 bis 6, **dadurch gekennzeichnet, daß** der wenigstens eine Polyoxyalkylen-Ester einer Hydroxyfettsäure ausgewählt ist unter die aus Solutol™ HS 15 (Polyethylenglykol 660-Hydroxystearat), Polyglykolestern von Polyethylenglykol und 12-Hydroxystearinsäure und Gemischen davon.

8. Pharmazeutische Formulierung nach einem der vorhergehenden Ansprüche, **dadurch gekennzeichnet, daß** der wenigstens eine Alkohol ausgewählt ist unter Ethanol, 1-Propanol, 2-Propanol und Gemische davon.

9. Pharmazeutische Formulierung nach einem der vorhergehenden Ansprüche, **dadurch gekennzeichnet, dass** das wenigstens eine Polyol ausgewählt ist unter Glycerin, Propylenglykol, 1,3-Butylenglykol und Gemische davon.

10. Pharmazeutische Formulierung nach einem der vorhergehenden Ansprüche, **dadurch gekennzeichnet, daß** die Formulierung eine Menge von Ketoprofen im Bereich von 2% bis 15% (w/v), bevorzugt zwischen 5% und 10% (w/v) und stärker bevorzugt von etwa 10% (w/v) enthält.

11. Pharmazeutische Formulierung nach einem der vorhergehenden Ansprüche, **dadurch gekennzeichnet, dass** die Formulierung eine Menge des wenigstens einen Polyoxyalkylen-Esters einer Hydroxyfettsäure im Bereich von 1% bis 20% (w/v), bevorzugt von 2% bis 15 % (w/v), stärker bevorzugt von 5% bis 15% (w/v) und am meisten bevorzugt von etwa 5% bis etwa 10% (w/v) enthält.

12. Pharmazeutische Formulierung nach einem der vorhergehenden Ansprüche, **dadurch gekennzeichnet, dass** die Formulierung eine Menge des wenigstens einen Alkohols im Bereich von 1 % bis 30% (w/v), bevorzugt von 2% bis 20% (w/v) enthält.

13. Pharmazeutische Formulierung nach einem der vorhergehenden Ansprüche, **dadurch gekennzeichnet, dass** die Formulierung eine Menge des wenigstens einen Polyols im Bereich von 1 % bis 30% (w/v), bevorzugt von 2% bis 20% (w/v) enthält.

14. Pharmazeutische Formulierung nach einem der vorhergehenden Ansprüche, **dadurch gekennzeichnet, daß** die Formulierung ein Gemisch aus wenigstens einem Alkohol und wenigstens einem Polyol enthält, wobei die Konzentration des wenigstens einen Alkohols im Bereich von 5% bis 15% (w/v) und die Konzentration des wenigstens einen Polyols im Bereich von 5% bis 15% (w/v) liegt.

15. Pharmazeutische Formulierung nach einem der vorhergehenden Ansprüche, **dadurch gekennzeichnet, dass** die Formulierung einen pH-Wert im Bereich von 6,5 bis 8,5, vorzugsweise von 7,0 bis 8,0 aufweist.

## Revendications

1. Formulation pharmaceutique comprenant (i) du kétoprofène, (ii) au moins un ester de polyoxyalkylène d'un acide gras hydroxylé, (iii) au moins un composé organique substitué par hydroxy choisi dans le groupe constitué d'au moins un alcool, au moins un polyol et des mélanges de ceux-ci, et (iv) de l'eau.

2. Formulation pharmaceutique selon la revendication 1, **caractérisée en ce que** ledit au moins un ester de polyoxyalkylène d'un acide gras hydroxylé est obtenu à partir de l'estérification d'un acide gras hydroxylé ayant de 8 à 30 atomes de carbone avec un polyoxyalkylène ayant un poids moléculaire dans la plage de 200 à 6 000.

3. Formulation pharmaceutique selon la revendication 2, **caractérisée en ce que** ledit acide gras hydroxylé a de 14 à 24 atomes de carbone, et ledit polyoxyalkylène a un poids moléculaire dans la plage de 400 à 1 500.

4. Formulation pharmaceutique selon l'une quelconque des revendications 2 et 3, **caractérisée en ce que** ledit acide gras hydroxylé est un acide gras hydroxylé saturé ou insaturé choisi dans le groupe constitué de l'acide hydroxycaprylique, l'acide hydroxycaprique, l'acide hydroxylaurique, l'acide hydroxymyristique, l'acide hydroxypalmitique, l'acide hydroxystéarique, l'acide hydroxyarachidique, l'acide hydroxybéhénique, l'acide hydroxylignocérique, l'acide hydroxymyristoléique, l'acide hydroxypalmitoléique, l'acide hydroxyoléique, l'acide hydroxylinoléique, l'acide hydroxylinolénique, l'acide hydroxyarachidonique, l'acide hydroxyeicosapentaénoique, l'acide hydroxyérucique, et l'acide hydroxydocosahexaénoïque.

5. Formulation pharmaceutique selon la revendication 4, **caractérisée en ce que** ledit acide gras hydroxylé est un acide gras hydroxylé saturé choisi dans le groupe constitué de l'acide hydroxylaurique, l'acide hydroxymyristique, l'acide hydroxypalmitique, l'acide hydroxystéarique, et l'acide hydroxyarachidique.

6. Formulation pharmaceutique selon l'une quelconque des revendications 2 à 5, **caractérisée en ce que** ledit polyoxyalkylène est choisi dans le groupe constitué de polyéthylèneglycol 200 (PEG 200), polyéthylèneglycol 300 (PEG 300), polyéthylèneglycol 400 (PEG 400), polyéthylèneglycol 600 (PEG 600), polyéthylèneglycol 660 (PEG 660), polyéthylèneglycol 1000 (PEG 1000), polyéthylèneglycol 1500 (PEG 1500), polyéthylèneglycol 3000 (PEG 3000), polyéthylèneglycol 3350 (PEG 3350), polyéthylèneglycol 4000 (PEG 4000), polyéthylèneglycol 6000 (PEG 6000), et des mélanges de ceux-ci.

7. Formulation pharmaceutique selon l'une quelconque des revendications 3 à 6, **caractérisée en ce que** ledit au moins un ester de polyoxyalkylène d'un acide gras hydroxylé est choisi dans le groupe de Solutol™ HS 15 (hydroxystéarate de polyéthylèneglycol 660), ester de polyglycol de polyéthylèneglycol et acide 12-hydroxystéarique, et des mélanges de ceux-ci.

8. Formulation pharmaceutique selon l'une quelconque des revendications précédentes, **caractérisée en ce que** ledit au moins un alcool est choisi dans le groupe comprenant éthanol, 1-propanol, 2-propanol, et un mélange de ceux-ci.

9. Formulation pharmaceutique selon l'une quelconque des revendications précédentes, **caractérisée en ce que** ledit au moins un polyol est choisi dans le groupe comprenant glycérol, propylèneglycol, 1,3-butylèneglycol, et des mélanges de ceux-ci.

10. Formulation pharmaceutique selon l'une quelconque des revendications précédentes, **caractérisée en ce que** ladite formulation comprend une quantité de kétoprofène dans la plage de 2 % à 15 % (m/v), de préférence entre 5 % et 10 % (m/v), et plus préférablement d'environ 10 % (m/v).

11. Formulation pharmaceutique selon l'une quelconque des revendications précédentes, **caractérisée en ce que** ladite formulation comprend une quantité dudit au moins un ester de polyoxyalkylène d'un acide gras hydroxylé dans la plage de 1 % à 20 % (m/v), de préférence de 2 % à 15 % (m/v), plus préférablement de 5 % à 15 % (m/v), et de manière préférée entre toutes d'environ 5 % à environ 10 % (m/v).

12. Formulation pharmaceutique selon l'une quelconque des revendications précédentes, **caractérisée en ce que** ladite formulation comprend une quantité dudit au moins un alcool dans la plage de 1 % à 30 % (m/v), de préférence de 2 % à 20 % (m/v).

13. Formulation pharmaceutique selon l'une quelconque des revendications précédentes, **caractérisée en ce que** ladite formulation comprend une quantité dudit au moins un polyol dans la plage de 1 % à 30 % (m/v), de préférence de 2 % à 20 % (m/v).

14. Formulation pharmaceutique selon l'une quelconque des revendications précédentes, **caractérisée en ce que** ladite formulation comprend un mélange d'au moins un alcool et au moins un polyol, la concentration dudit au moins un alcool est dans la plage de 5 % à 15 % (m/v) et la concentration dudit au moins un polyol est dans la plage de 5 % à 15 % (m/v).

15. Formulation pharmaceutique selon l'une quelconque des revendications précédentes, **caractérisée en ce que** ladite formulation a une valeur de pH dans la plage de 6,5 à 8,5, de préférence de 7,0 à 8,0.
